# DEMANDE DE BREVET EUROPEEN

(11) **EP 3 973 853 A1**
(43) Date de publication de la demande: **30.03.2022**
(21) Numéro de dépôt: 20198909.2
(22) Date de dépôt: 29.09.2020
(51) Int. Cl.: A61B 5/00, A61B 5/11

(54) **SYSTÈMES DE CAPTATION DE LA DÉGLUTITION ET D'ANALYSE À DISTANCE COMPRENANT UNE CAPTURE DE MOUVEMENTS**

(71) Demandeur: Swallis Médical, 67000 Strasbourg (FR)
(72) Inventeur: PERRIN, Nicolas, 67120 Kolbsheim (FR); NICOLINI, Linda, 31200 Toulouse (FR); NEVEU, Fabrice, 34000 Montpellier (FR)
(74) Mandataire: Cabinet Camus Lebkiri

(57) **Abrégé**

Un aspect de l'invention concerne un système de captation de la déglutition d'au moins un utilisateur, le système étant caractérisé en ce qu'il comprend :
- Au moins un dispositif de détection de la déglutition comprenant au moins un capteur de détection de déglutition configuré pour acquérir au moins un signal de déglutition de l'utilisateur,
- Au moins un système de capture de mouvements de l'utilisateur configuré pour acquérir au moins un signal de mouvement de l'utilisateur pendant la captation du au moins un signal de déglutition de l'utilisateur par le capteur du dispositif de détection de la déglutition.

## Description

### DOMAINE TECHNIQUE DE L'INVENTION

Le domaine technique de l'invention est celui de la détection de la déglutition.

La présente invention concerne un système de captation de la déglutition et d'analyse à distance de la déglutition comprenant une capture de mouvements, et en particulier une capture de mouvements utilisant au moins une caméra.

### ARRIERE-PLAN TECHNOLOGIQUE DE L'INVENTION

Les patients atteints de troubles de la déglutition, aussi appelés « dysphagie », présentent des difficultés à avaler les aliments, par exemple le manque de coordination dans le cheminement des aliments entre la bouche et l'estomac passant par le pharynx et l'œsophage, et des risques de fausses-routes. Concernant les fausses-routes, on parle de pénétration si l'aliment entre dans le larynx mais reste au-dessus de la glotte, des cordes vocales, et d'aspiration s'il passe les cordes vocales. L'aspiration entraîne une des réponses corporelles, normalement une toux, mais s'il existe un trouble de sensibilité, l'aspiration peut être sans toux et donc silencieuse.

La dysphagie peut par exemple apparaître chez des patients après un accident vasculaire cérébral (« AVC »), après un traumatisme crânio-cérébral (« TCC »), après une Sclérose Latérale Amiotrophique ou chez les patients atteints de la maladie d'Alzheimer ou de maladie neurodégénératives.

Afin de détecter le niveau de dysphagie d'un patient, par exemple pour évaluer le risque de fausse route, le type, la cause et/ou le niveau de gravité de la dysphagie, de nombreuses méthodes d'examen ont été développées.

L'étude de l'accélérométrie de la déglutition a notamment été développée face à un besoin de méthodes non-invasives d'évaluation de la dysphagie, particulièrement grâce à la miniaturisation et à l'amélioration de la précision des accéléromètres électroniques. Des dispositifs colliers comprenant un accéléromètre ont été développés pour l'acquisition de signaux d'accélérométrie de déglutition au niveau du larynx. En effet, une déglutition peut être divisée en trois phases :
- la phase orale de la déglutition est volontaire et comprend le mouvement postérieur de la langue et de l'os hyoïde,
- la phase laryngo-pharyngée est automatique et réflexe et comprend le mouvement laryngé, l'élévation de l'os hyoïde, la fermeture de l'épiglotte et le passage du bolus vers l'orifice œsophagien, et
- la phase œsophagienne est réflexe et comprend la contraction péristaltique, le repositionnement de l'os hyoïde et du larynx et la réouverture de l'épiglotte.

La captation de ces signaux permet leur traitement par ordinateur et leur caractérisation par rapport à ces trois phases de la déglutition, et des techniques de classification automatique ont ainsi été appliquées à ces signaux dans l'état de la technique pour la détection de dysphagies, d'aspirations, de fausses-routes silencieuses et/ou pour l'évaluation du niveau de dysphagie.

De tels dispositifs colliers peuvent en outre comprendre un capteur de son laryngé, par exemple un microphone, pour la mesure du son de déglutition, par exemple afin de filtrer et d'analyser les sons de respiration, de toux et les sons liés à la voix du patient. Le microphone peut aussi permettre la détection de la déglutition, par exemple en détectant le son d'ascension laryngé correspondant à l'ascension du larynx quand le bolus est localisé dans l'oropharynx et/ou l'hypopharynx, le son d'ouverture du sphincter supérieur correspondant au transit du bolus à travers le sphincter supérieur, et le son de relâchement laryngé correspondant à la descente et à l'ouverture du larynx quand le bolus a atteint l'œsophage, comme décrit par Sylvain Morinière et al. dans « Origin of the Sound Components During Pharyngeal Swallowing in Normal Subjects », Dysphagia, 2008.

Ces dispositifs colliers peuvent être utilisés pour la détection et l'analyse de déglutitions chez un patient atteint de dysphagie, ou pour l'analyse des exercices de rééducation à la déglutition. Dans la détection et l'analyse de déglutitions, un utilisateur porte un dispositif collier pour lequel les signaux sont analysés généralement par un dispositif connecté au collier tel qu'un smartphone ou un autre dispositif électronique spécialisé. Pour l'analyse des données et la réalisation d'exercices, les patients utilisateurs de tels dispositifs doivent tout de même se rendre chez un spécialiste, notamment car les seules données récupérées par le capteur ne permettent ni une analyse précise et complète de la déglutition ni de faire un retour à l'utilisateur sur sa façon de déglutir pour lui proposer d'éventuels exercices. En outre, bien que les signaux mesurés par les accéléromètres et/ou microphones permettent une caractérisation de la déglutition, cette caractérisation ne permet pas de remonter à l'origine du problème de déglutition, c'est-à-dire à sa cause, mais seulement d'indiquer si la déglutition était bonne ou mauvaise.

Il existe donc un besoin de pouvoir étudier la déglutition d'un patient de manière complète et précise.

### RESUME DE L'INVENTION

L'invention offre une solution aux problèmes évoqués précédemment, en permettant une étude de la déglutition complète et précise, permettant de faire de la rééducation, ne nécessitant pas un déplacement chez le praticien au moment de la mesure des signaux par un dispositif de détection.

Un aspect de l'invention concerne un système de captation de la déglutition d'au moins un utilisateur, le système étant caractérisé en ce qu'il comprend :
- Au moins un dispositif de détection de la déglutition comprenant au moins un capteur de détection de déglutition configuré pour acquérir au moins un signal de déglutition de l'utilisateur,
- Au moins un système de capture de mouvements de l'utilisateur configuré pour acquérir au moins un signal de mouvement de l'utilisateur pendant la captation du au moins un signal de déglutition de l'utilisateur par le capteur du dispositif de détection de la déglutition.

Grâce à l'invention, il est possible d'étudier l'ensemble de la déglutition et d'avoir une vision extérieure objective de la déglutition. Il est ainsi possible, grâce à la présence d'un système de capture de mouvements de l'utilisateur, d'associer un ou des mouvements de l'utilisateur à la déglutition mesurée par un capteur de détection de la déglutition, et donc de remonter à une potentielle cause d'une mauvaise déglutition, qui peut être une mauvaise posture ou un mauvais mouvement.

En mesurant conjointement la déglutition par un accéléromètre ou un microphone au niveau du larynx et les mouvements par un système de capture de mouvements de l'utilisateur, la captation de la déglutition est enrichie et il est possible de remonter à la cause d'une mauvaise déglutition et d'analyser a posteriori les mouvements de l'utilisateur qui ont conduit au résultat de la caractérisation de la déglutition.

Outre les caractéristiques qui viennent d'être évoquées dans le paragraphe précédent, le système de captation selon un aspect de l'invention peut présenter une ou plusieurs caractéristiques complémentaires parmi les suivantes, considérées individuellement ou selon toutes les combinaisons techniquement possibles :
- le système de capture de mouvements comprend au moins une caméra,
- le système de capture de mouvements comprend en outre au moins un marqueur de capture de mouvement en coopération avec la au moins une caméra,
- la au moins une caméra est configurée pour acquérir des images de la tête, du cou et du thorax de l'utilisateur,
- le système de captation de la déglutition comprend en outre deux caméras, une caméra parmi les deux caméras étant configurée pour acquérir des images de face de l'utilisateur et une autre caméra parmi les deux caméras étant configurée pour obtenir des images de profil de l'utilisateur,
- le capteur de détection de déglutition est un microphone pour détecter un son de déglutition et/ou un accéléromètre pour détecter une vibration de déglutition,
- le dispositif de détection de la déglutition comprend en outre au moins un capteur parmi les capteurs de fréquence cardiaque, de température du corps, de sudation, de son de respiration, de fréquence respiratoire, d'activité musculaire,
- le système de captation de la déglutition comprend en outre un module réseau configuré pour envoyer à travers un réseau le au moins un signal de déglutition de l'utilisateur et le au moins un signal de mouvement de l'utilisateur pendant la captation du au moins un signal de déglutition de l'utilisateur,
- le système de captation de la déglutition comprend en outre un processeur configuré pour caractériser la déglutition de l'utilisateur à partir du signal de déglutition de l'utilisateur et du signal de mouvement de l'utilisateur.

Un autre aspect de l'invention concerne un système d'analyse de la déglutition d'au moins un utilisateur comprenant :
- le système de captation de la déglutition de l'utilisateur selon l'invention et
- un dispositif d'analyse à distance comprenant :
   - un module réseau configuré pour recevoir du module réseau du système de captation, via le réseau, le au moins un signal de déglutition de l'utilisateur et le au moins un signal de mouvement de l'utilisateur pendant la captation du au moins un signal de déglutition de l'utilisateur et
   - un module d'affichage configuré pour afficher simultanément le au moins un signal de déglutition de l'utilisateur et le au moins un signal de mouvement de l'utilisateur pendant la captation du au moins un signal de déglutition de l'utilisateur.

Outre les caractéristiques qui viennent d'être évoquées dans le paragraphe précédent, le système d'analyse selon un aspect de l'invention peut présenter une ou plusieurs caractéristiques complémentaires parmi les suivantes, considérées individuellement ou selon toutes les combinaisons techniquement possibles :
- le dispositif d'analyse à distance comprend un processeur configuré pour caractériser la déglutition de l'utilisateur à partir du signal de déglutition de l'utilisateur,
- la caractérisation de la déglutition par le processeur comprend une classification du signal de déglutition,
- le processeur est configuré pour caractériser la déglutition de l'utilisateur en utilisant en outre le signal de mouvement de l'utilisateur.

Le système d'analyse selon l'invention permet de ne pas nécessiter un déplacement des patients auprès de leur praticien pour analyser leur déglutition. Cela permet aussi des analyses de la déglutition en direct, en ayant accès à toutes les informations nécessaires pour l'analyse de la déglutition à distance : un accès aux informations de mesure de signaux de la déglutition grâce aux capteurs de détection de la déglutition ainsi qu'un accès aux informations visuelles grâce aux caméras, l'accès à ces informations étant simultané grâce au module d'affichage du système d'analyse. Cet affichage simultané permet de mettre en relation les deux types d'informations et de travailler à distance comme si l'utilisateur était physiquement dans la même pièce que le praticien.

En outre, la caractérisation de la déglutition peut être réalisée à distance par le dispositif d'analyse à distance, permettant de réduire le coût des dispositifs de détection de la déglutition en réduisant le besoin de puissance de calcul au niveau des dispositifs de détection de la déglutition et en déplaçant la puissance de calcul dans un dispositif distant, accessible et utilisable par un praticien par exemple.

Encore un autre aspect de l'invention concerne un procédé de captation de la déglutition d'au moins un utilisateur par un système de captation de la déglutition selon l'invention, le procédé comprenant les étapes de :
- Captation d'au moins un signal de déglutition par un dispositif de détection de déglutition du système de captation de la déglutition ;
- Acquisition par le système de capture de mouvements du signal de mouvement de l'utilisateur simultanément à la captation du signal de déglutition par le dispositif de détection de la déglutition.

Encore un autre aspect de l'invention concerne un procédé d'analyse de la déglutition d'au moins un utilisateur par un système d'analyse de la déglutition selon l'invention, le procédé comprenant les étapes de :
- Réception, par le dispositif d'analyse à distance, du au moins un signal de déglutition et du au moins un signal de mouvement de l'utilisateur acquis simultanément à la captation du signal de déglutition;
- caractérisation de la déglutition par le processeur du système d'analyse de la déglutition à partir du signal de mouvement et du signal de déglutition reçus.

L'étape de caractérisation de la déglutition du procédé d'analyse de la déglutition d'un utilisateur par un système d'analyse de la déglutition selon l'invention peut comprendre la classification du signal de mouvement et du signal de déglutition reçus.

Le système d'analyse selon l'invention trouve un intérêt particulier pour les utilisateurs pouvant difficilement se déplacer, dans les cas d'interdictions de déplacements, et de manière générale pour limiter les déplacements du praticien et des utilisateurs.

L'invention et ses différentes applications seront mieux comprises à la lecture de la description qui suit et à l'examen des figures qui l'accompagnent.

### BREVE DESCRIPTION DES FIGURES

Les figures sont présentées à titre indicatif et nullement limitatif de l'invention.
- La figure 1 montre une représentation schématique d'un système de captation de la déglutition d'au moins un utilisateur selon l'invention.
- La figure 2 montre une représentation schématique d'un système d'analyse à distance de la déglutition d'au moins un utilisateur selon un premier mode de réalisation de l'invention.
- La figure 3 montre une représentation schématique d'un système d'analyse à distance de la déglutition d'au moins un utilisateur selon un deuxième mode de réalisation de l'invention.
- La figure 4 montre une représentation schématique d'un procédé de captation de la déglutition d'au moins un utilisateur selon l'invention,
- La figure 5 montre une représentation schématique d'un procédé d'analyse de la déglutition d'au moins un utilisateur selon l'invention.

### DESCRIPTION DETAILLEE

Les figures sont présentées à titre indicatif et nullement limitatif de l'invention.

Sauf précision contraire, un même élément apparaissant sur des figures différentes présente une référence unique.

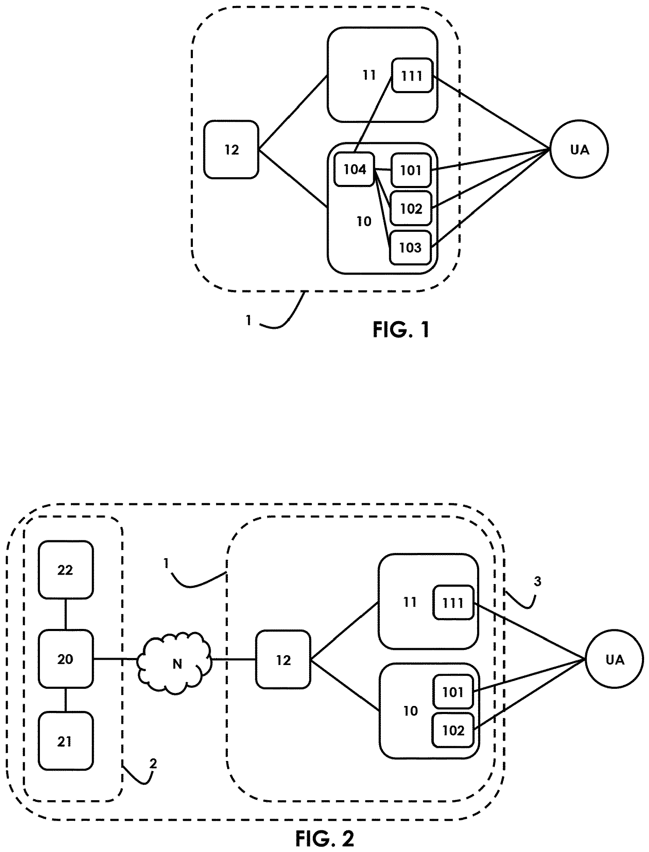

La [Fig. 1] montre une représentation schématique d'un système de captation de la déglutition d'au moins un utilisateur selon l'invention.

Le système de captation 1 représenté à la [Fig.1] permet d'acquérir au moins un signal de déglutition et au moins un signal de mouvement d'un utilisateur UA. La présente invention couvre aussi les cas où le système de captation 1 permet d'acquérir au moins un signal de déglutition et au moins un signal de mouvement d'une pluralité d'utilisateurs (non représenté).

Le système de captation 1 selon l'invention comprend au moins un dispositif de détection de la déglutition 11 comprenant au moins un capteur 111 de détection de déglutition configuré pour acquérir au moins un signal de déglutition de l'utilisateur UA. Le dispositif de détection de la déglutition 11 est préférentiellement un dispositif de type collier, porté autour du cou, de manière connue de l'homme du métier. Le dispositif de détection de la déglutition 11 peut cependant être tout type de dispositif permettant la détection de la déglutition.

Le capteur de détection de déglutition 111 du dispositif de détection de la déglutition 11 peut par exemple être un accéléromètre situé au niveau du larynx de l'utilisateur UA. Il peut alors mesurer un signal de déglutition de l'utilisateur UA, par exemple un signal de mouvement laryngé correspondant à une déglutition ou tout autre mouvement permettant de caractériser une déglutition. Le capteur de détection de déglutition 111 peut par exemple être un microphone, le signal de déglutition de l'utilisateur mesuré étant alors un son laryngé, ou tout autre son permettant de caractériser une déglutition. Le capteur de détection de déglutition 111 peut être tout capteur capable de mesurer un signal de déglutition permettant de caractériser une déglutition de l'utilisateur. En outre, le dispositif de détection de la déglutition 11 peut comprendre une pluralité de capteurs de détection de déglutition 111 (non représenté), par exemple une combinaison d'un microphone et d'un accéléromètre pour améliorer la précision et la fiabilité de la détection de la déglutition.

Le dispositif de détection de la déglutition 11 peut aussi comprendre au moins un capteur parmi les capteurs de fréquence cardiaque, de température du corps, de sudation, de son de respiration, de fréquence respiratoire, d'activité musculaire (non représentés). Le dispositif de détection de la déglutition 11 représenté à la [Fig. 1] peut comprendre tous les capteurs précédemment cités, mais il peut aussi ne comprendre qu'un des capteurs précédemment cités ou toute combinaison possible des capteurs précédemment cités. Les capteurs précédemment cités permettent de connaître précisément l'état de l'utilisateur UA pendant un exercice de rééducation à la déglutition ou pendant un examen de la déglutition.

Le système de captation 1 selon l'invention comprend en outre au moins un système 10 de capture de mouvements de l'utilisateur UA configuré pour acquérir au moins un signal de mouvement de l'utilisateur UA pendant la captation du au moins un signal de déglutition de l'utilisateur UA par le capteur 111 du dispositif 11 de détection de la déglutition. Cela permet au système de captation 1 d'être adaptable aux dispositifs de détection de la déglutition existants, permettant, par un ajout notamment du système 10 de capture de mouvements, d'avoir un système permettant la captation simultanée de plusieurs types de signaux, et notamment de signaux de déglutition et de signaux de mouvement de l'utilisateur UA.

Le système 10 de capture de mouvements de l'utilisateur UA selon l'invention comprend au moins un, préférentiellement deux capteurs de mouvements 101 et 102 de l'utilisateur UA. Ainsi, le système 10 de capture de mouvements de l'utilisateur UA peut ne comprendre que le capteur 101, deux capteurs 101 et 102, ou plus de deux capteurs. On entend par « capteur de mouvement » tout capteur permettant de transformer un mouvement de l'utilisateur en signal analogique ou numérique. Ainsi, les capteurs 101 et 102 peuvent être des caméras, ou une association de marqueurs optiques avec des caméras, ou tout autre capteur permettant de transformer un mouvement de l'utilisateur en signal analogique ou numérique. Bien entendu, un capteur de mouvements au sens de la présente invention permet aussi d'acquérir des données de position lorsque l'utilisateur UA n'est pas en mouvement. Ainsi, une unique image provenant d'une caméra permet d'accéder à des données de position de l'utilisateur UA sans qu'il n'y ait de mouvement de la part de l'utilisateur. En fonction du système, un marqueur optique et deux caméras permettent aussi d'avoir accès à une position, par exemple par triangulation, sans qu'il n'y ait forcément de mouvement de la part de l'utilisateur.

Le système 10 de capture de mouvements de l'utilisateur UA selon l'invention est configuré pour acquérir au moins un signal de mouvement de l'utilisateur UA pendant la captation du au moins un signal de déglutition de l'utilisateur UA par le capteur 111 du dispositif 11 de détection de la déglutition. On entend par « signal de mouvement» de l'utilisateur UA tout signal permettant de restituer un mouvement, une posture ou un changement de posture de l'utilisateur UA, c'est-à-dire tout signal représentant un mouvement, une posture ou un changement de posture de l'utilisateur UA, tel qu'une image ou une suite d'images (vidéo), une représentation en deux ou trois dimensions de points particuliers ou de membres ou ensemble de membres de l'utilisateurs UA, un signal électrique proportionnel à une grandeur mesurée, ou tout autre signal permettant la représentation ou la restitution de mouvement(s), de la posture ou d'un changement de posture de l'utilisateur UA.

On entend par « mouvement » de l'utilisateur tout changement de position de l'utilisateur UA et plus précisément tout changement de position d'un membre ou d'un ensemble de membres de l'utilisateur par rapport à un autre ou par rapport à un repère, par exemple tout changement de position de la tête par rapport au cou et/ou par rapport au thorax et/ou par rapport à un repère fixe ou mobile. En outre, un signal indiquant qu'aucun mouvement n'a eu lieu est un signal de mouvement, car l'information qu'aucun mouvement n'a eu lieu est une information importante concernant les mouvements de l'utilisateur UA. Plus particulièrement, toute succession d'images de l'ensemble tête, cou et thorax de l'utilisateur UA ou de toute combinaison de ces trois membres ou ensembles de membres est comprise dans l'appellation « signal de mouvement » de l'utilisateur UA. Préférentiellement, le système 10 de capture de mouvements de l'utilisateur UA permet de capturer un changement de posture de l'utilisateur UA.

Le système 10 de capture de mouvements de l'utilisateur UA comprend ainsi au moins un capteur 101, par exemple une caméra. Le système 10 de capture de mouvements de l'utilisateur UA peut comprendre en outre un deuxième capteur 102, par exemple une autre caméra, configurée pour acquérir des images vues d'un autre angle par rapport à la première caméra 101. Par exemple, la caméra 101 est configurée pour acquérir des images de face de l'utilisateur UA et la caméra 102 est configurée pour obtenir des images de profil de l'utilisateur UA. Lorsque l'on mentionne qu'une caméra est « configurée pour acquérir » des images particulières, on entend que la caméra est orientée et/ou positionnée et/ou paramétrée de façon à capturer ces images particulières. Dans un autre mode de réalisation, le capteur 101 peut être une caméra et le capteur 102 peut être un capteur infrarouge ou un projecteur infrarouge en coopération avec la caméra 101. Les capteurs 101 et 102 peuvent être tout type de capteur permettant de capturer un signal de mouvement de l'utilisateur UA, préférentiellement de mouvement au niveau du larynx ou de l'ensemble tête-couthorax. Lorsque les capteurs 101 et 102 sont des caméras, ou lorsque l'un au moins des capteurs 101 ou 102 est une caméra, le système 10 de capture de mouvements de l'utilisateur UA selon l'invention peut mettre en œuvre un floutage adaptatif du visage de l'utilisateur UA afin de conserver l'anonymat de l'utilisateur UA.

Préférentiellement, le système 10 de capture de mouvements de l'utilisateur UA comprend un marqueur de capture de mouvement 103 en coopération avec au moins un des deux capteurs 101 ou 102, préférentiellement en coopération avec les deux capteurs 101 et 102. Un tel marqueur de capture de mouvements 103 permet de capturer les mouvements de l'utilisateur avec plus de précision qu'en utilisant uniquement des caméras et en réalisant de la reconnaissance de formes ou de mouvements à partir des images capturées. Le marqueur de capture de mouvements 103 est porté par l'utilisateur UA, soit directement collé, attaché ou accroché à sa peau ou à un vêtement qu'il porte, soit collé, attaché ou accroché au dispositif de détection de la déglutition 10 qu'il porte. Un marqueur de capture de mouvements 103 peut par exemple être un marqueur optique passif, réfléchissant par exemple un rayonnement infrarouge émis par les caméras 101 et 102 et capté en retour par ces mêmes caméras 101 et 102. Une seule caméra 101 peut être utilisée, mais l'invention est préférentiellement mise en œuvre avec deux caméras 101 et 102. Un marqueur de capture de mouvements 103 peut aussi par exemple être un marqueur optique actif, émettant une lumière ou un rayonnement infrarouge en direction de caméras ou de capteurs photosensibles. Le marqueur de capture de mouvement 103 peut en outre être tout type de marqueur permettant la capture de mouvements de l'utilisateur UA.

Le signal de mouvement acquis par le système de capture de mouvements 10 est acquis pendant la captation du au moins un signal de déglutition de l'utilisateur par le capteur 111 du dispositif de détection de la déglutition 11. Pour cela, le système de capture de mouvements 10 comprend au moins un processeur 104 configuré pour synchroniser les captations de signaux des deux types. Ainsi, il est possible de mettre en lien les mouvements de l'utilisateur UA et sa déglutition. Le processeur 104 peut par exemple être configuré pour transmettre un ordre de mesure de mouvements aux capteurs 101 et 102 du système de capture de mouvements 10 lorsqu'il détecte que le dispositif de détection de la déglutition 11 mesure un signal de déglutition de l'utilisateur UA. Pour cela, le processeur 104 peut être directement relié au capteur de détection de la déglutition 111. Le processeur met en œuvre des instructions stockées par une mémoire du système de capture de mouvements 10 (non représentée) qui, lorsqu'elles sont exécutées par le processeurs 104, le conduisent à mettre en œuvre la synchronisation de la mesure des signaux de déglutitions et de mouvement de l'utilisateur UA. Dans un autre mode de réalisation non représenté, le processeur peut ne pas faire partie intégrante du système de capture de mouvements 10, mais peut par exemple faire partie du dispositif de détection de la déglutition 11, ou être extérieur au dispositif de détection de la déglutition 11 et au système de capture de mouvements 10. Préférentiellement, le processeur 104 est compris dans le système de capture de mouvements 10 afin de pouvoir simplement ajouter ce système de capture de mouvements 10 à un dispositif de détection de la déglutition 11 existant pour améliorer la détection et l'analyse de la déglutition.

Le système de captation de la déglutition 1 selon l'invention peut comprendre en outre un module réseau 12, comme représenté à la [Fig. 1]. Ce module réseau 12 peut permettre d'envoyer les signaux de déglutition et de mouvement à travers un réseau, à un autre dispositif d'analyse décrit plus tard. L'invention couvre aussi les systèmes de captation de la déglutition 1 ne comprenant pas de module réseau 12. Le module réseau 12 permet avantageusement de pouvoir analyser à distance les signaux captés par le système de capture de mouvements 10 et par le dispositif de détection de la déglutition 11. Le module réseau 12 peut permettre de communiquer à travers un ou plusieurs réseaux, de même type ou de différents types. Par exemple, le module réseau 12 peut permettre de communiquer à travers un réseau internet, par exemple en utilisant la suite des protocoles TCP/IP. Le module réseau 12 peut permettre de communiquer via une connexion sans-fil, par exemple en Wi-Fi, ou via une connexion filaire, par exemple en Ethernet.

La [Fig. 2] montre une représentation schématique d'un système d'analyse à distance de la déglutition d'au moins un utilisateur selon un premier mode de réalisation d'un second aspect de l'invention.

En effet, un second aspect de l'invention concerne un système d'analyse à distance 3 de la déglutition d'un utilisateur UA, le système d'analyse à distance 3 comprenant le système de captation 1 de la déglutition d'un utilisateur UA tel que présenté précédemment et un dispositif d'analyse à distance 2. Le système de captation 1 de la déglutition d'un utilisateur UA et le dispositif d'analyse à distance 2 sont reliés et communiquent à travers un réseau N. Comme indiqué précédemment, ce réseau N peut être tout réseau de communication, préférentiellement le réseau internet. Pour communiquer à travers le réseau N, le système de captation 1 de la déglutition d'un utilisateur UA comprend un module réseau 12 et le dispositif d'analyse à distance 2 comprend un module réseau 20. Les deux modules réseau 12 et 20 peuvent être identiques ou différents, et doivent chacun pouvoir permettre de communiquer à travers le réseau N. Le module réseau 20 peut permettre de communiquer à travers un ou plusieurs réseaux, de même type ou de différents types. Par exemple, le module réseau 20 peut permettre de communiquer à travers un réseau internet, par exemple en utilisant la suite des protocoles TCP/IP. Le module réseau 20 peut permettre de communiquer via une connexion sans-fil, par exemple en Wi-Fi, ou via une connexion filaire, par exemple en Ethernet. Le module réseau 20 est configuré pour recevoir du module réseau 12 du système de captation 1, via le réseau N, le au moins un signal de déglutition de l'utilisateur UA mesurée par le capteur 111 du dispositif de détection de la déglutition 11 et le au moins un signal de mouvement de l'utilisateur capté par le système de capture de mouvement 10 pendant la captation du au moins un signal de déglutition de l'utilisateur UA. Ainsi, le dispositif d'analyse à distance reçoit conjointement les deux signaux mesurés pendant la même période, permettant une analyse des deux signaux pour caractériser la déglutition de l'utilisateur UA.

Le dispositif d'analyse à distance 2 comprend en outre un module d'affichage 21 configuré pour afficher simultanément le au moins un signal de déglutition de l'utilisateur UA et le au moins un signal de mouvement de l'utilisateur UA pendant la captation du au moins un signal de déglutition de l'utilisateur UA. Le module d'affichage 21 peut être un écran ou tout autre type de dispositif permettant d'afficher des données à un utilisateur du dispositif d'analyse à distance 2 tel qu'un praticien.

Le dispositif d'analyse à distance 2, dans un mode de réalisation préférentiel et comme représenté à la [Fig. 2], comprend en outre un processeur 22, configuré pour caractériser la déglutition de l'utilisateur UA à partir des signaux reçus du système de captation de la déglutition 1. On entend par « caractériser » le fait de donner un attribut à une déglutition représentée par un signal. On entendra aussi par « caractériser » un signal le fait d'associer un attribut d'une déglutition à un signal représentant cette déglutition. Un exemple de caractérisation, qui est aussi un mode de réalisation préférentiel, est la classification d'un signal, par exemple dans une classe correspondant à une déglutition correcte ou dans une classe correspondant à une déglutition incorrecte. Cette classification peut être réalisée de manière connue de l'homme du métier par un algorithme d'apprentissage automatique utilisant un modèle statistique ou encore un réseau de neurones. Les signaux issus du capteur 111 peuvent permettre au modèle de classification d'être plus précis et plus fiable dans ses prises de décision pour caractériser un signal de déglutition comme étant « incorrect », c'est-à-dire représentatif d'une dysphagie de l'utilisateur étudié, par exemple une déglutition présentant un risque de fausse-route ou d'aspiration, ou « correct », c'est-à-dire représentatif d'une déglutition non caractéristique d'une dysphagie de l'utilisateur étudié. Le processeur 22 peut ne prendre en compte que les signaux de déglutition issus du capteur 111 du dispositif de détection de la déglutition 11, ou peut prendre en compte conjointement les signaux de déglutition issus du capteur 111 du dispositif de détection de la déglutition 11 et les signaux de mouvement issus du système de capture de mouvements 10. Cela peut permettre notamment une meilleure caractérisation de la déglutition.

Préférentiellement, les signaux de déglutition, les signaux de mouvement et leur caractérisation attribuée par le processeur 22 sont affichés conjointement par le module d'affichage 21. Préférentiellement encore, les signaux de déglutition et de mouvement reçus peuvent être affichés en temps réel ou en quasi-temps-réel, c'est-à-dire par exemple dès leur réception par le module de réception 20, après caractérisation de l'un au moins de ces signaux par le processeur 22. Les signaux de déglutition et de mouvement reçus peuvent être affichés sans leur caractérisation, par exemple avant que la caractérisation ait été réalisée par le processeur 22, et la caractérisation peut être affichée par la suite par le module d'affichage 21, lorsqu'elle est disponible, c'est-à-dire lorsqu'elle a été réalisée par le processeur 22. Préférentiellement encore, le dispositif d'analyse à distance comprend un module de stockage, configuré pour stocker les signaux de déglutition et de mouvement reçus et la caractérisation de la déglutition représentée par ces signaux. Le module de stockage peut aussi être accessible à travers un réseau.

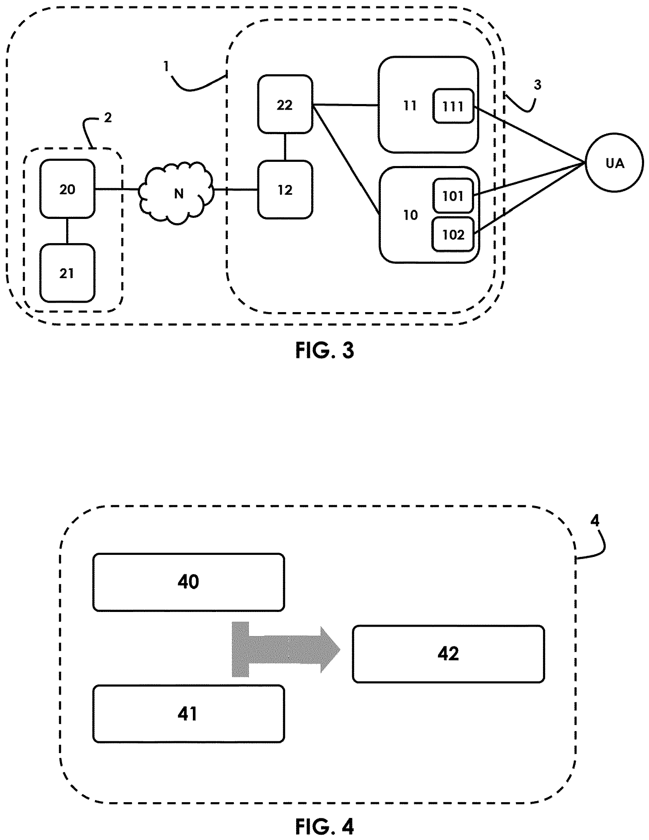

La [Fig. 3] montre une représentation schématique d'un système d'analyse à distance de la déglutition d'au moins un utilisateur selon un deuxième mode de réalisation de l'invention.

Dans le deuxième mode de réalisation représenté à la [Fig. 3], le dispositif d'analyse à distance ne comprend pas de processeur 22. Le système de captation 1 de la déglutition comprend le processeur 22. La caractérisation des signaux de déglutition et/ou des signaux de mouvement est alors réalisée au niveau du système de captation 1. Ainsi, seule la caractérisation de la déglutition et les signaux de mouvement peuvent être envoyés au dispositif 2 d'analyse à distance à travers le réseau N, pour analyse par un praticien ou un utilisateur grâce au module d'affichage 21. Alternativement, le signal de détection de la déglutition peut être envoyé, avec le signal de mouvement et la caractérisation de la déglutition, ou toute combinaison possible et intéressante de ces trois informations. Préférentiellement, l'utilisateur du dispositif d'analyse à distance 2 peut analyser visuellement grâce au module d'affichage 2 les signaux de déglutition et de mouvement ainsi que la caractérisation de la déglutition associée conjointement, c'est-à-dire sur le même écran ou sur le même affichage.

Ainsi, un utilisateur du dispositif d'analyse à distance 2, par exemple un praticien, peut réaliser des consultations à distance (aussi appelées « téléconsultations »), en ayant accès à un maximum d'informations et notamment à des images du patient ou de l'utilisateur UA déglutissant conjointement aux signaux de déglutition correspondants.

Le dispositif d'analyse à distance 2 peut en outre permettre d'analyser les signaux de déglutitions et de mouvement reçus d'une façon plus poussée que par un simple affichage, par exemple en utilisant des outils d'analyse et de traitement de signaux et/ou d'analyse et de traitement d'image. Pour cela, le dispositif d'analyse à distance 2 peut en outre comprendre une interface homme-machine (non représentée).

La [Fig. 4] montre une représentation schématique d'un procédé de captation de la déglutition d'au moins un utilisateur par un système de captation de la déglutition selon l'invention.

Le procédé 4 de captation de la déglutition d'au moins un utilisateur UA comprend trois étapes 40 à 42.

Dans une première étape 40 au moins un signal de déglutition est capté par un dispositif de détection de déglutition 11 du système de captation de la déglutition 1 tel que présenté précédemment. Cette étape de captation comprend une mesure, par un capteur 111 de déglutition du dispositif de détection de la déglutition 11 présenté précédemment, d'au moins un signal associé à une déglutition, par exemple un son ou une accélérométrie.

Une deuxième étape 41 comprend une acquisition par le système de capture de mouvements 10 d'un signal de mouvement de l'utilisateur UA simultanément à la captation du signal de déglutition par le dispositif de détection de la déglutition. Comme expliqué précédemment, la synchronisation des étapes 40 et 41 peut être réalisée en utilisant un processeur 104, ou par tout autre moyen permettant la synchronisation de la mesure des deux signaux.

Le procédé 4 de captation de la déglutition d'au moins un utilisateur UA comprend préférentiellement une étape 42 d'envoi du signal de déglutition et du signal de mouvement à travers un réseau N. Cet envoi est réalisé par le module réseau 12 du système de captation de la déglutition 1.

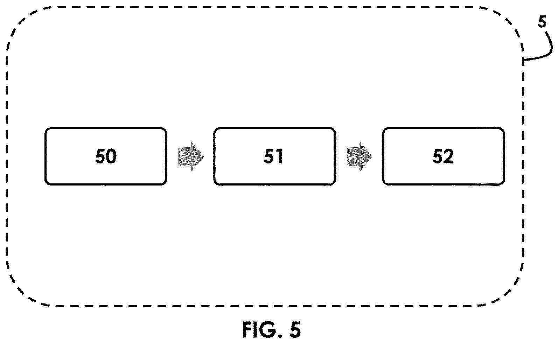

La [Fig. 5] montre une représentation schématique d'un procédé d'analyse à distance de la déglutition d'au moins un utilisateur par un système d'analyse de la déglutition selon l'invention.

Le procédé 5 d'analyse à distance de la déglutition d'au moins un utilisateur UA par le système d'analyse de la déglutition 3 tel que présenté précédemment comprend trois étapes 50 à 52.

Dans une première étape 50, le dispositif d'analyse à distance 2 reçoit, à travers le réseau N, le au moins un signal de déglutition et le au moins un signal de mouvement de l'utilisateur UA acquis simultanément tel que décrit dans le procédé 4 de captation de la déglutition.

Le procédé 5 d'analyse à distance de la déglutition comprend ensuite une deuxième étape 51 de caractérisation de la déglutition par le processeur 22 du système d'analyse de la déglutition 2 à partir du signal de mouvement et du signal de déglutition reçus. Comme décrit précédemment, cette étape 51 de caractérisation du signal de déglutition et du signal de mouvement reçus peut comprendre par exemple la classification du signal de mouvement et du signal de déglutition reçus.

Le procédé 5 d'analyse à distance de la déglutition comprend préférentiellement une troisième étape d'affichage, par le module d'affichage 21, des signaux de déglutition et de mouvement reçus et de la caractérisation réalisée à l'étape 51 du procédé 5. Comme expliqué précédemment, l'affichage de la caractérisation peut comprendre l'affichage de l'attribut donné aux signaux ou à la déglutition par le processeur 22 lors de l'étape 51 de caractérisation.

## Revendications

1. Système de captation de la déglutition d'au moins un utilisateur, le système étant **caractérisé en ce qu'**il comprend :
- Au moins un dispositif de détection de la déglutition comprenant au moins un capteur de détection de déglutition configuré pour acquérir au moins un signal de déglutition de l'utilisateur,
- Au moins un système de capture de mouvements de l'utilisateur configuré pour acquérir au moins un signal de mouvement de l'utilisateur pendant la captation du au moins un signal de déglutition de l'utilisateur par le capteur du dispositif de détection de la déglutition.

2. Système de captation de la déglutition selon la revendication précédente **caractérisé en ce que** le système de capture de mouvements comprend au moins une caméra.

3. Système de captation de la déglutition selon la revendication précédente **caractérisé en ce que** le système de capture de mouvements comprend en outre au moins un marqueur de capture de mouvement en coopération avec la au moins une caméra.

4. Système de captation de la déglutition selon l'une quelconque des revendications 2 ou 3 **caractérisé en ce que** la au moins une caméra est configurée pour acquérir des images de la tête, du cou et du thorax de l'utilisateur.

5. Système de captation de la déglutition selon l'une quelconque des revendications 2 à 4 **caractérisé en ce qu'**il comprend deux caméras, une caméra parmi les deux caméras étant configurée pour acquérir des images de face de l'utilisateur et une autre caméra parmi les deux caméras étant configurée pour obtenir des images de profil de l'utilisateur.

6. Système de captation de la déglutition selon l'une quelconque des revendications précédentes **caractérisé en ce que** le capteur de détection de déglutition est un microphone pour détecter un son de déglutition et/ou un accéléromètre pour détecter une vibration de déglutition.

7. Système de captation de la déglutition selon l'une quelconque des revendications précédentes **caractérisé en ce que** le dispositif de détection de la déglutition comprend en outre au moins un capteur parmi les capteurs de fréquence cardiaque, de température du corps, de sudation, de son de respiration, de fréquence respiratoire, d'activité musculaire.

8. Système de captation de la déglutition selon l'une quelconque des revendications précédentes **caractérisé en ce qu'**il comprend en outre un module réseau configuré pour envoyer à travers un réseau le au moins un signal de déglutition de l'utilisateur et le au moins un signal de mouvement de l'utilisateur pendant la captation du au moins un signal de déglutition de l'utilisateur.

9. Système de captation de la déglutition selon l'une quelconque des revendications précédentes **caractérisé en ce qu'**il comprend en outre un processeur configuré pour caractériser la déglutition de l'utilisateur à partir du signal de déglutition de l'utilisateur et du signal de mouvement de l'utilisateur.

10. Système d'analyse de la déglutition d'au moins un utilisateur comprenant :
- le système de captation de la déglutition de l'utilisateur selon la revendication 8 et
- un dispositif d'analyse à distance comprenant :
∘ un module réseau configuré pour recevoir du module réseau du système de captation, via le réseau, le au moins un signal de déglutition de l'utilisateur et le au moins un signal de mouvement de l'utilisateur pendant la captation du au moins un signal de déglutition de l'utilisateur et
∘ un module d'affichage configuré pour afficher simultanément le au moins un signal de déglutition de l'utilisateur et le au moins un signal de mouvement de l'utilisateur pendant la captation du au moins un signal de déglutition de l'utilisateur.

11. Système d'analyse de la déglutition selon la revendication précédente **caractérisé en ce que** le dispositif d'analyse à distance comprend un processeur configuré pour caractériser la déglutition de l'utilisateur à partir du signal de déglutition de l'utilisateur.

12. Système d'analyse de la déglutition selon la revendication précédente **caractérisé en ce que** la caractérisation de la déglutition par le processeur comprend une classification du signal de déglutition.

13. Système d'analyse de la déglutition selon l'une quelconque des revendications 10 ou 11 **caractérisé en ce que** le processeur est configuré pour caractériser la déglutition de l'utilisateur en utilisant en outre le signal de mouvement de l'utilisateur.

14. Procédé de captation de la déglutition d'au moins un utilisateur par un système de captation de la déglutition selon l'une quelconque des revendications 1 à 6, le procédé comprenant les étapes de :
- Captation d'au moins un signal de déglutition par un dispositif de détection de déglutition du système de captation de la déglutition ;
- Acquisition par le système de capture de mouvements du signal de mouvement de l'utilisateur simultanément à la captation du signal de déglutition par le dispositif de détection de la déglutition.

15. Procédé d'analyse de la déglutition d'au moins un utilisateur par un système d'analyse de la déglutition selon l'une quelconque des revendications 11 à 13, le procédé comprenant les étapes de :
- Réception, par le dispositif d'analyse à distance, du au moins un signal de déglutition et du au moins un signal de mouvement de l'utilisateur acquis simultanément à la captation du signal de déglutition;
- caractérisation de la déglutition par le processeur du système d'analyse de la déglutition à partir du signal de mouvement et du signal de déglutition reçus.

16. Procédé d'analyse de la déglutition selon la revendication précédente **caractérisé en ce que** l'étape de caractérisation de la déglutition comprend la classification du signal de mouvement et du signal de déglutition reçus.
